# FASCICULE DE BREVET EUROPEEN

(11) **EP 2 142 917 B1**
(45) Date de publication et mention de la délivrance du brevet: **01.06.2016**
(21) Numéro de dépôt: 08736391.7
(22) Date de dépôt: 18.04.2008
(51) Int. Cl.: G01N 27/22, A47J 37/12, G01N 33/03

(54) **APPAREIL DE CUISSON COMPORTANT UN DISPOSITIF DE MESURE CAPACITIVE DE LA QUALITE ET/OU DEGRADATION D'UN FLUIDE ET SON UTILISATION**
KOCHGERÄT MIT EINER KAPAZITIVEN MESSVORRICHTUNG ZUR BESTIMMUNG DER QUALITÄT UND/ODER VERSCHLECHTERUNG EINES FLUIDES UND ENTSPRECHENDE VERWENDUNG
COOKING APPARATUS COMPRISING A DEVICE FOR THE CAPACITIVE MEASUREMENT OF THE QUALITY AND/OR DEGRADATION OF A FLUID AND CORRESPONDING USE

(30) Priorité: 20.04.2007 CH 654072007
(43) Date de publication de la demande: 13.01.2010
(73) Titulaire: Alpsens Technologies Inc., 1024 Ecublens (CH)
(72) Inventeur: CHAMBON, Gérald, CH-1018 Lausanne (CH)
(74) Mandataire: Ravenel, Thierry Gérard Louis
(86) Numéro de dépôt international: PCT/EP2008/054746
(87) Numéro de publication internationale: WO 2008/135368

(56) Documents cités:
- EP-A- 1 393 664
- EP-A- 1 588 158
- US-B1- 6 777 009

## Description

La présente invention concerne un dispositif de mesure capacitive de la qualité et/ou de la dégradation d'un fluide, notamment d'une huile. L'invention concerne en particulier un tel dispositif comportant un capteur capacitif permettant la mesure de la qualité et/ou de la dégradation d'une huile de friture, directement disposé dans l'appareil de cuisson, et dans lequel le capteur capacitif est orienté d'une manière telle qu'il améliore de façon importante la qualité et la fiabilité de la mesure capacitive.

Il est bien connu que les huiles alimentaires se dégradent lors de la cuisson, notamment lorsqu'elles sont portées de manière répétée à des températures élevées. Typiquement, pour frire des aliments ces huiles sont portées à des températures de l'ordre de 180°C. A ces températures se produisent une multitude de réactions chimiques, telles que des polymérisations, des thermo-oxydations, etc., qui altèrent de manière importante la qualité de l'huile. La quantité de certains produits des ces réactions ne doit pas dépasser un seuil imposé par la législation, car au-delà de ce seuil l'huile est considérée comme impropre à la consommation. Il est donc important de pouvoir détecter ce seuil de manière fiable afin de remplacer l'huile dès que cela est nécessaire. Pendant longtemps ce moment a été laissé à l'appréciation des cuisiniers qui suite à une inspection visuelle et/ou olfactive déterminaient si l'huile était encore propre à la consommation. Bien entendu un telle méthode est purement subjective et n'est par conséquent pas fiable.

Un dispositif de mesure capacitive de la qualité et/ou de la dégradation d'une huile de friture a été proposé le brevet EP 1 588 158 pour remédier à ces inconvénients.

Dans ce dispositif le capteur capacitif est directement disposé dans la cuve de l'appareil de cuisson, ce capteur étant encapsulé dans un boîtier de protection perforé fixé dans une zone immergée de la cuve.

Bien que le dispositif décrit dans cette demande de brevet fonctionne de manière satisfaisante il n'en demeure pas moins que la mesure de capacité à l'intérieur d'une friteuse est une opération très sensible

Au cours de ses recherches, le demandeur a découvert que le positionnement ou l'orientation du capteur à l'intérieur de la cuve est cruciale pour l'obtention d'une mesure capacitive fiable. En effet tant l'eau, que les impuretés de l'huile et les gradients de température présents dans la cuve ont un effet important sur la mesure et la précision de cette dernière.

L'eau, principalement présente dans l'huile neuve et dans les aliments à frire, se place au fond de la cuve lorsque la température de l'huile est inférieure à 100°C. Lorsque l'huile monte en température, il se crée une zone « chaude » au dessus du corps de chauffe (env 180°C) et éventuellement une zone froide (env. 80°C) en dessous du corps de chauffe si la friteuse est prévue pour cet effet. L'eau générée par la cuisson peut rester fractionner avec l'huile lorsque sa température est inférieure à 100 °C.

Lorsque l'on cuit un aliment, de l'eau est rejeté du à la chimie de la cuisson. Une partie de cette eau passe de l'état liquide à l'état de vapeur, ce qui va produire un brassage naturel ce qui va également faire remonter l'eau qui se trouvait en bas de la cuve. Un brassage mécanique est souvent réalisé par l'opérateur afin de mieux cuire les aliments.

C'est alors que des gouttes d'eau ou de la vapeur d'eau peuvent venir se bloquer dans le capteur ou à sa surface, la valeur mesurée devient inutilisable.

En outre, les impuretés, principalement constituées de panures et de morceaux d'aliments frits, provoquent l'encrassement du capteur et ce principalement sur la surface sensible du capteur et sur toute partie fermée de l'encapsulation. Ces impuretés ont donc également tendance à dégrader la qualité des mesures.

La mesure de capacité est encore fortement influencée par les gradients de température présents dans la cuve. Lorsqu'une friteuse est allumée, la température au-dessus du corps de chauffe atteint environ 180°C. Sous le corps de chauffe, la température augmente lentement en l'absence de brassage par insertion de produits à frire ce qui poussent l'huile chaude du haut vers le bas. Ce brassage de l'huile provoque des différences de température à la surface sensible du capteur. Ce gradient thermique peut être très marqué et les variations de températures très rapides lorsqu'on plonge des produits à frire dans la cuve.

Cette température influence très fortement la constante diélectrique de l'huile, donc sa capacité, qui en l'occurrence diminue. La mesure de température, et principalement son point de mesure, constitue donc aussi un élément essentiel pour la précision du système.

L'invention a donc pour but de remédier à ce problème en fournissant un appareil de cuisson défini par la revendication 1 du brevet. L'invention a également pour objet l'utilisation d'un dispositif de mesure capacitive de la qualité et/ou la dégradation d'un fluide définie par la revendication 7 du brevet.

Selon une caractéristique avantageuse le plan des électrodes du capteur forme un angle compris entre 0° et 30° avec la direction verticale.

Selon une caractéristique préférée plan des électrodes du capteur forme un angle sensiblement nulle avec la direction verticale.

Selon une autre caractéristique du dispositif le capteur est fixé dans la cuve de manière amovible sur un support de fixation.

Selon encore une autre caractéristique du dispositif le capteur le capteur est fixé directement sous le corps de chauffe de l'appareil de cuisson.

L'orientation du capteur encapsulé minimise les effets du gradient de température : on admet l'hypothèse que l'huile est composée d'isothermes (même profondeur dans la cuve = même température).

Le capteur encapsulé est placé « sur la tranche » dans la friteuse (côté étroit pointant vers le haut) afin de minimiser les surfaces planes où panures et eau pourraient s'accumuler.

La structure du capteur ainsi que son encapsulation sont ouvertes au maximum permettant un flux d'huile naturel. Ce flux garantit un « nettoyage » permanent du capteur et élimine les accumulations.

Selon un mode de réalisation une sonde de température est associée au capteur et est située sur la surface du capteur, la partie sensible contre l'extérieur. Cette position permet de mesurer la température de l'huile au plus près de la surface du capteur.

Le capteur encapsulé est amovible. Il est donc possible de l'enlever de la cuve afin de le nettoyer au cas où l'entretien préventif des friteuses (nettoyage, boil-out, ...) ne suffit pas.

Grâce à ces caractéristiques on peut garantir un fonctionnement optimal du capteur et améliorer la précision de la mesure capacitive, de manière simple et efficace. Un avantage important étant de s'affranchir des principaux problèmes inhérents à toute friteuse par une position réfléchie et un design adéquat lors du développement et de l'installation.

D'autres caractéristiques et avantages de la présente invention apparaîtront dans la description suivante d'un mode de réalisation préféré d'un dispositif de mesure selon l'invention, présenté à titre d'exemple non limitatif en référence aux dessins annexés, dans lesquels :
- La figure 1 est une vue schématique en coupe d'une cuve de friteuse d'un premier mode de réalisation un dispositif de mesure avec son capteur encapsulé orienté dans la cuve selon l'enseignement de l'invention;
- La figure 2 est une vue schématique en bout du dispositif représenté à la figure 1,
- La figure 3 est une vue schématique de dessus du dispositif représenté à la figure 1 ;
- La figure 4 est une vue schématique en coupe d'une cuve de friteuse d'un deuxième mode de réalisation un dispositif de mesure avec son capteur encapsulé orienté dans la cuve selon l'enseignement de l'invention
- La figure 5 est une vue schématique en bout du dispositif représenté à la figure 4,
- La figure 6 est une vue schématique de dessus du dispositif représenté à la figure 4 ;

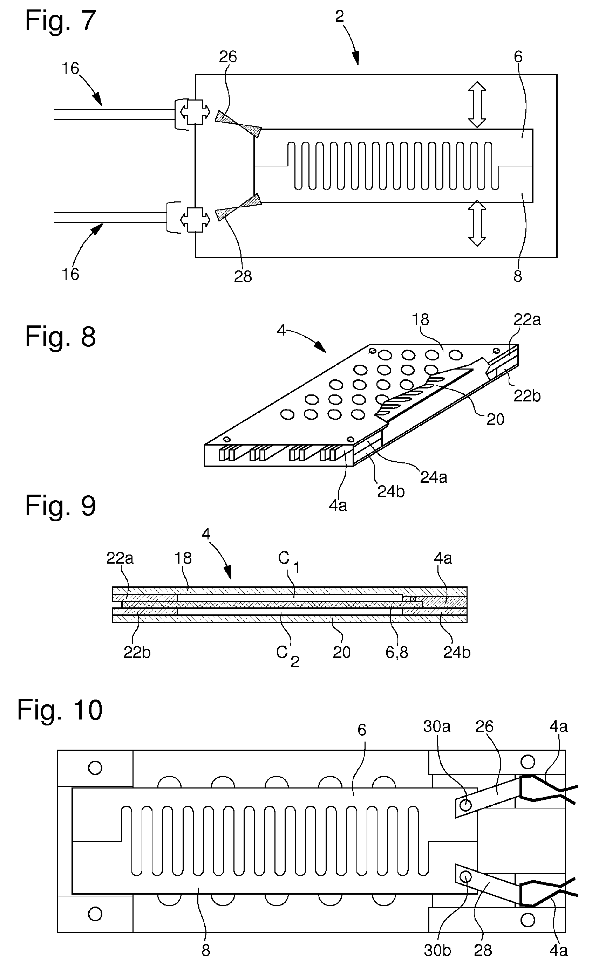

- La figure 7 est une vue schématique du capteur encapsulé du dispositif selon l'invention
- La figure 8 est une vue schématique en perspective du capteur encapsulé du dispositif selon l'invention
- La figure 9 est une vue schématique en coupe du capteur encapsulé selon l'invention; et
- La figure 10 est une vue schématique en élévation du capteur non encapsulé selon l'invention;

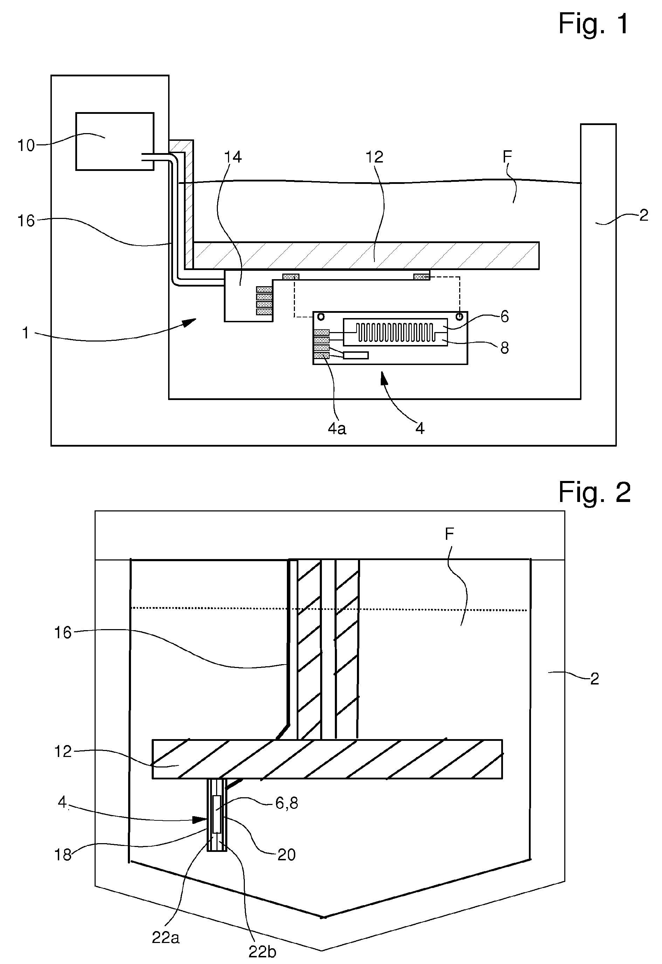

En se référant aux figures 1 et 2, on voit un mode de réalisation d'un dispositif de mesure capacitive de la qualité et/ou de la dégradation d'un fluide notamment d'une huile, désigné par la référence générale 1.

On notera que la description qui suit sera faite dans une application du dispositif 1 à la mesure de la qualité et/ou de la dégradation d'une huile alimentaire ou analogue, utilisée pour frire des aliments dans un appareil de cuisson comprenant une cuve 2 dans laquelle l'huile peut être chauffée typiquement jusqu'à environ 200°C.

Le dispositif de mesure 1 comprend un capteur encapsulé 4 comportant une paire d'électrodes 6, 8 espacées l'une de l'autre et destinées à être immergées dans un fluide F (Figure 2), par exemple l'huile d'une friteuse, dont on souhaite mesurer la qualité et/ou la dégradation et déterminer si elle est toujours propre à l'emploi. Les électrodes 6, 8 forment avec l'huile F un élément capacitif de mesure EFM dont la capacité varie en fonction de la constante diélectrique de l'huile. Lorsque l'huile se dégrade, la quantité de composés polaires présents dans celle-ci augmente et conduit à une augmentation de sa constante diélectrique. Ainsi, en mesurant l'évolution de la capacité de l'élément capacitif de mesure EFM, on peut déterminer le degré de qualité et/ou de dégradation de l'huile. Le capteur 4, et plus précisément son élément capacitif EFM, est donc capable de fournir un signal électrique de sortie représentatif de la constante diélectrique de l'huile sur une large plage de températures, en particulier entre 20°C et 200°C. Le signal électrique est traité par un circuit de traitement électronique 10 disposé à l'extérieur de la cuve 2. Le capteur 4 est relié au circuit de traitement électronique par des contacts électriques 4a. Le capteur est par exemple fixé de façon amovible sous le corps de chauffe 12 par l'intermédiaire d'un support de fixation et de connexion 14 solidaire de ce dernier. Typiquement le capteur 4 peut s'enficher dans le support 14 par ses contacts électriques 4 qui sont du par exemple conformée en pince élastiques. Le support de fixation et de connexion 14 est relié au circuit électronique au moyen de câbles 16 protégés, par exemple dans des tubes.

Chaque électrode 6, 8 de la paire présente la forme d'un peigne ayant une pluralité de dents 6a, 8a sensiblement parallèles entre elles et s'étendant à partir d'une base 6b, 8b. Les électrodes 6 et 8 sont disposées l'une par rapport à l'autre de sorte que les dents 6a d'une électrode 6 sont imbriquées entre les dents 8a de l'autre électrode 8. Les dents des électrodes 6 et 8 sont ainsi disposées sensiblement dans un même plan.

On notera à ce propos que les électrodes 6 et 8 sont par exemple formées à partir d'une même plaque plane découpée de façon appropriée, la plaque étant suffisamment rigide pour que les électrodes gardent leur forme lorsqu'elles sont manipulées. Dans l'exemple décrit, les électrodes sont réalisées à partir d'une plaque en un acier alimentaire (acier inox austénitique de type 18-10 à bas carbone) ayant une épaisseur comprise entre 0,1 et 3 mm. D'autres types d'aciers alimentaires peuvent être également utilisés par exemple le Z7CN18-09, le Z3CND18-12-02, Z6CNDT17-12 et Z7CNU16-04. La plaque est découpée au moyen d'un faisceau laser, ce qui permet de réaliser entre les dents des électrodes des entrefers compris entre 10 nm et 1 mm. Il est bien entendu que plus l'entrefer est faible, plus la sensibilité de l'élément capacitif est grande. Selon une variante de réalisation, on peut également envisager de réalisé des électrodes formées d'un substrat enrobé d'un matériau conducteur, par exemple un substrat enrobé d'une couche d'or de platine ou analogue.

Les électrodes 6 et 8 sont disposées dans un boîtier d'encapsulation perforé. Ce boîtier est formée de plaques 18, 29 planes perforées métalliques entre lesquelles s'étendent les électrodes 6, 8 avec interposition aux extrémités de deux paires d'entretoises 22a, 22b et 24a, 24b en matière isolante entre lesquelles les électrodes 6, 8 formant la sonde impédimétrique sont prises en sandwich. Les électrodes 6, 8 sont fixées aux plaques 18, 20 via les entretoises 22a, 22b à une extrémité et sont guidées libres dans les entretoises 24a, 24b à leur extrémité opposée.

Les perforations des plaques 18, 20 de l'encapsulation sont disposées en regard de la région de mesure des électrodes 6 et 8, c'est-à-dire en regard des entrefers définis par les espaces entre les dents 6a de l'électrode 6 et les dents 8a de l'électrode 8. Grâce à cette configuration, le fluide à mesurer, en l'occurrence l'huile, baigne les deux faces des électrodes 6 et 8 de part et d'autre du plan de ces électrodes de sorte qu'elle peut venir circuler au voisinage des dents 6a et 8a des électrodes 6 et 8.

Cette structure d'encapsulation des électrodes permet d'optimiser la circulation de l'huile autour des deux faces des électrodes planes et notamment de créer deux canaux C1, C2 définis respectivement entre une première surface des électrodes 6, 8 et la plaque perforée 18 et une deuxième surface des électrodes 6, 8 opposée à la première et la plaque perforée 20.

La fixation des électrodes 6 et 8 aux entretoises est réalisée par des moyens élastiques, à savoir par deux lames ressort 26, 28 assurant également la fonction de contact électrique entre les électrodes et des éléments de contact 4a du capteur 2.

Par cette fixation élastique on réalise un découplage mécanique du capteur de son boîtier d'encapsulation. Ces lames, découpées dans une feuille d'acier inox de 100 microns d'épaisseur par électro-érosion, assurent le positionnement de la sonde dans le boîtier d'encapsulation. La sonde est guidée dans la direction perpendiculaire au plan des plaques 18 et 20 par ses boulons de fixation qui se logent dans des alésages des entretoises 24a, 24b. Un petit jeu est laissé afin que la sonde soit « libre » dan son emplacement, reposant simplement sur les pièces isolantes.

Les entretoises sont réalisées de préférence en un matériau résistant à des températures comprises entre 20°C et 200°C et présentant un faible coefficient de dilatation thermique, tel qu'un matériau céramique. Cependant elles peuvent être réalisées en tout autre matériau isolant compatible avec l'application du dispositif de mesure envisagée. A titre d'exemple pour une application alimentaire devant être stable dans la gamme de température susmentionnée, les entretoises pourraient également être réalisées en un polymère fluoré tel que le Téflon.

Le capteur encapsulé est orienté dans la cuve de manière que l'axe longitudinal de la base de chaque électrode s'étend parallèlement au fond de la cuve. Le plan des électrodes du capteur forme un angle sensiblement nul avec la direction verticale.

Il est toutefois bien entendu que le plan des électrodes du capteur peut former également un angle compris entre 0° et 60° avec la direction verticale. L'angle limite étant déterminé de manière qu'il permette une circulation d'un flux d'huile autour de la sonde impédimétrique, à savoir à travers les canaux C1 et C2 sans que les impuretés, principalement constituées de panures et de morceaux d'aliments frits viennent adhérer à la surface de la sonde.

Selon le mode de réalisation illustré aux figures 1 à 3, le capteur est fixé dans la cuve de manière amovible sur un support de fixation est fixé directement sous le corps de chauffe de l'appareil de cuisson figure 1.

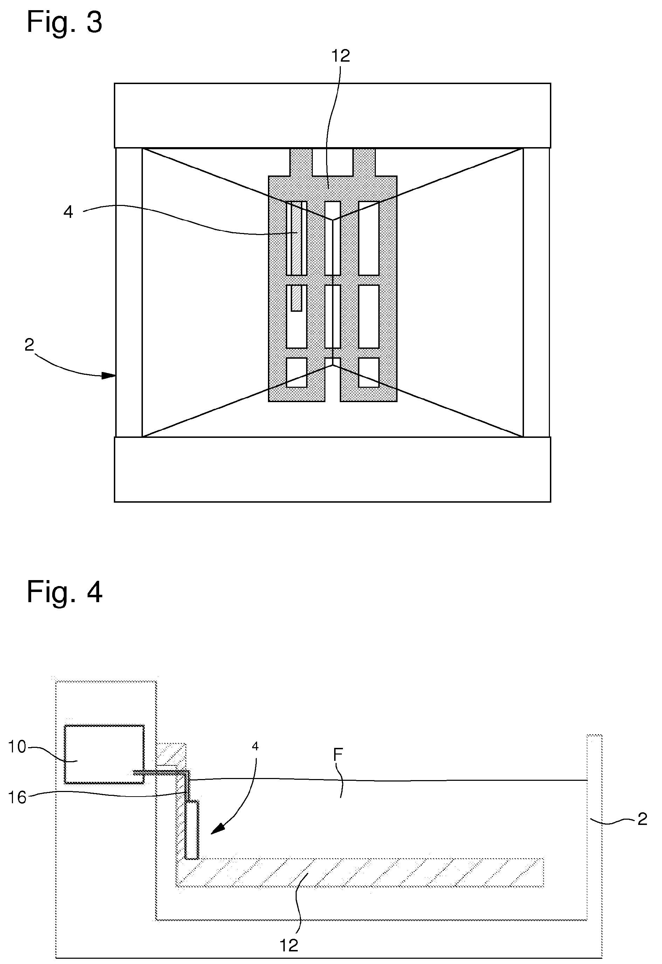

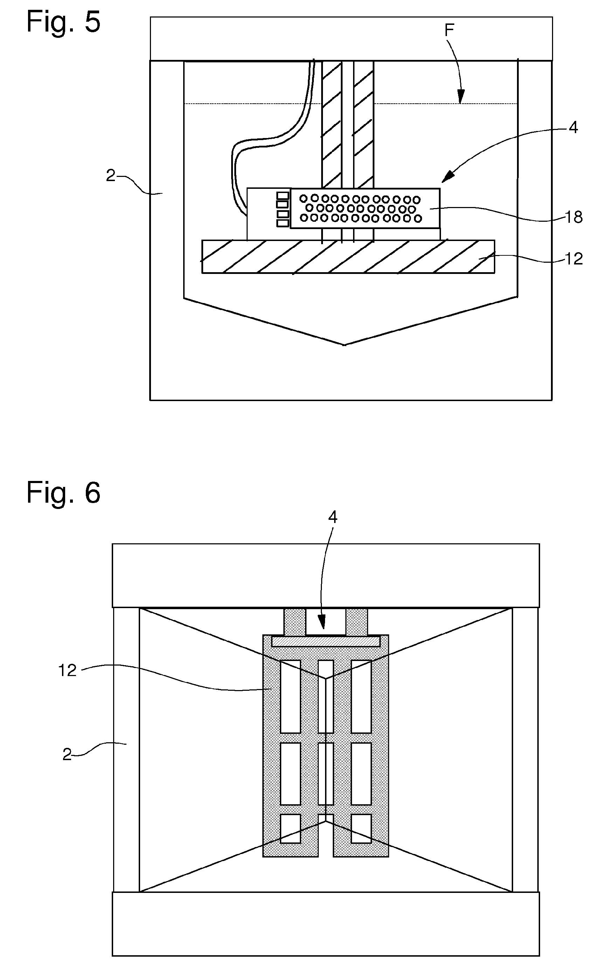

Selon le mode de réalisation illustré aux figures 4 à 6, le capteur est fixé sur la partie de fixation du corps de chauffe s'étendant parallèlement aux parois verticales de la cuve.

## Revendications

1. Appareil de cuisson comportant une cuve et un dispositif de mesure capacitive de la qualité et/ou de la dégradation d'un fluide comportant un capteur capacitif encapsulé dans un boîtier perforé et fixé dans la cuve de l'appareil de cuisson comprenant un fond, ledit capteur étant relié à un circuit électronique de traitement, lequel capteur comprend une paires d'électrodes planes ayant chacune la forme d'un peigne ayant une pluralité de dents sensiblement parallèles entre elles et s'étendant à partir d'une base, les électrodes étant disposées l'une par rapport à l'autre de sorte que les dents d'une électrode sont imbriquées entre les dents de l'autre électrode sensiblement dans un même plan **caractérisé en ce que** le capteur encapsulé est orienté dans la cuve de manière que l'axe longitudinal de la base de chaque électrode s'étend parallèlement au fond de la cuve et **en ce que** le plan des électrodes du capteur forme un angle compris entre 0° et 60° avec la direction verticale

2. Appareil de cuisson selon la revendication 1, **caractérisé en ce que** le plan des électrodes du capteur forme un angle compris entre 0° et 30° avec la direction verticale.

3. Appareil de cuisson selon la revendication 2 **caractérisé en ce que** le plan des électrodes du capteur forme un angle sensiblement nulle avec la direction verticale.

4. Appareil de cuisson selon l'une des revendications précédentes **caractérisé en ce que** le capteur est fixé dans la cuve de manière amovible sur un support de fixation.

5. Appareil de cuisson selon l'une des revendications précédentes **caractérisé en ce que** le capteur est fixé directement sous le corps de chauffe de l'appareil de cuisson.

6. Appareil de cuisson selon l'une des revendications 1 à 5 **caractérisé en ce que** le capteur est fixé sur la partie de fixation du corps de chauffe s'étendant parallèlement aux parois verticales de la cuve.

7. Utilisation d'un dispositif de mesure capacitive de la qualité et/ou de la dégradation d'un fluide comportant un capteur capacitif encapsulé dans un boîtier perforé et fixé dans la cuve d'un appareil de cuisson comprenant un fond, ledit capteur étant relié à un circuit électronique de traitement, lequel le capteur comprend une paires d'électrodes planes ayant chacune la forme d'un peigne ayant une pluralité de dents sensiblement parallèles entre elles et s'étendant à partir d'une base, les électrodes étant disposées l'une par rapport à l'autre de sorte que les dents d'une électrode sont imbriquées entre les dents de l'autre électrode sensiblement dans un même plan **caractérisé en ce que** le capteur encapsulé est orienté dans la cuve de manière que l'axe longitudinal de la base de chaque électrode s'étend parallèlement au fond de la cuve et **en ce que** le plan des électrodes du capteur forme un angle compris entre 0° et 60° avec la direction verticale

8. Utilisation du dispositif de mesure selon la revendication 7, **caractérisé en ce que** le plan des électrodes du capteur forme un angle compris entre 0° et 30° avec la direction verticale.

9. Utilisation du dispositif de mesure selon la revendication 8, **caractérisé en ce que** le plan des électrodes du capteur forme un angle sensiblement nulle avec la direction verticale.

10. Utilisation du dispositif de mesure selon l'une des revendications 7 à 9 **caractérisé en ce que** le capteur est fixé dans la cuve de manière amovible sur un support de fixation.

11. Utilisation du dispositif de mesure selon l'une des revendications 7 à 10 **caractérisé en ce que** le capteur est fixé directement sous le corps de chauffe de l'appareil de cuisson.

## Patentansprüche

1. Gargerät, umfassend einen Behälter und eine kapazitive Messvorrichtung der Qualität und/oder der Degeneration eines Fluids, umfassend einen in ein perforiertes Gehäuse eingekapselten und in dem Behälter des Gargeräts befestigten kapazitiven Sensor, umfassend einen Boden, wobei der Sensor mit einem elektronischen Verarbeitungskreis verbunden ist, wobei der Sensor ein Paar ebener Elektronen umfasst, die jeweils die Form eines Kamms haben, der eine Vielzahl zueinander etwa paralleler Zinken hat, die sich ab einer Basis erstrecken, wobei die Elektroden im Verhältnis zueinander derart angeordnet sind, dass sich die Zinken einer Elektrode zwischen den Zinken der anderen Elektrode etwa in einer selben Ebene verzahnen, **dadurch gekennzeichnet, dass** der eingekapselte Sensor im Behälter derart ausgerichtet ist, dass sich die Längsachse der Basis jeder Elektrode parallel zum Boden des Behälters erstreckt und dass die Ebene der Elektroden des Sensors einen Winkel zwischen 0° und 60° mit der Vertikalen bildet.

2. Gargerät nach Anspruch 1, **dadurch gekennzeichnet, dass** die Ebene der Elektroden des Sensors einen Winkel zwischen 0° und 30° mit der Vertikalen bildet.

3. Gargerät nach Anspruch 2, **dadurch gekennzeichnet, dass** die Ebene der Elektroden des Sensors einen Winkel von etwa Null mit der Vertikalen bildet.

4. Gargerät nach einem der vorangehenden Ansprüche, **dadurch gekennzeichnet, dass** der Sensor im Behälter lösbar auf einer Befestigungsunterlage befestigt ist.

5. Gargerät nach einem der vorangehenden Ansprüche, **dadurch gekennzeichnet, dass** der Sensor direkt unter dem Heizkörper des Gargeräts befestigt ist.

6. Gargerät nach einem der Ansprüche 1 bis 5, **dadurch gekennzeichnet, dass** der Sensor auf dem Befestigungsabschnitt des Heizkörpers befestigt ist, der sich parallel zu den vertikalen Wänden des Behälters erstreckt.

7. Verwendung einer kapazitiven Messvorrichtung der Qualität und/oder der Degeneration eines Fluids, umfassend einen in ein perforiertes Gehäuse eingekapselten und in dem Behälter des Gargeräts befestigten kapazitiven Sensor, umfassend einen Boden, wobei der Sensor mit einem elektronischen Verarbeitungskreis verbunden ist, wobei der Sensor ein Paar ebener Elektronen umfasst, die jeweils die Form eines Kamms haben, der eine Vielzahl zueinander etwa paralleler Zinken hat, die sich ab einer Basis erstrecken, wobei die Elektroden im Verhältnis zueinander derart angeordnet sind, dass sich die Zinken einer Elektrode zwischen den Zinken der anderen Elektrode etwa in einer selben Ebene verzahnen, **dadurch gekennzeichnet, dass** der eingekapselte Sensor im Behälter derart ausgerichtet ist, dass sich die Längsachse der Basis jeder Elektrode parallel zum Boden des Behälters erstreckt und dass die Ebene der Elektroden des Sensors einen Winkel zwischen 0° und 60° mit der Vertikalen bildet.

8. Verwendung der Messvorrichtung nach Anspruch 7, **dadurch gekennzeichnet, dass** die Ebene der Elektroden des Sensors einen Winkel zwischen 0° und 30° mit der Vertikalen bildet.

9. Verwendung der Messvorrichtung nach Anspruch 8, **dadurch gekennzeichnet, dass** die Ebene der Elektroden des Sensors einen Winkel von etwa Null mit der Vertikalen bildet.

10. Verwendung der Messvorrichtung nach einem der Ansprüche 7 bis 9, **dadurch gekennzeichnet, dass** der Sensor im Behälter auf einer Befestigungsunterlage lösbar befestigt ist.

11. Verwendung der Messvorrichtung nach einem der Ansprüche 7 bis 10, **dadurch gekennzeichnet, dass** der Sensor direkt unter dem Heizkörper des Gargeräts befestigt ist.

## Claims

1. Cooking apparatus including a vat and a device for the capacitive measurement of the quality and/or deterioration of a fluid that includes a sensor encapsulated in a perforated case and secured in the vat of a cooking apparatus, which includes a bottom, wherein said sensor is connected to an electronic processing circuit, wherein the sensor includes a pair of flat electrodes that each have the shape of a comb with a plurality of teeth, which are approximately parallel to each other and extend from a base, wherein the electrodes are arranged in relation to each other such that the teeth of one electrode fit between the teeth of the other electrode in approximately the same plane, **characterized in that** the encapsulated sensor is oriented in the vat such that the longitudinal axis of the base of each electrode extends parallel to the bottom of the vat and **in that** the plane of the sensor electrodes form an angle of between 0° and 60° with respect to the vertical direction.

2. Cooking apparatus according to claim 1, **characterized in that** the plane of the sensor electrodes forms an angle of between 0° and 30° with respect to the vertical direction.

3. Cooking apparatus according to claim 2, **characterized in that** the plane of the sensor electrodes forms an angle of approximately zero degrees with respect to the vertical direction.

4. Cooking apparatus according to any of the preceding claims, **characterized in that** the sensor is fixed in the vat in a removable manner on a securing support.

5. Cooking apparatus according to any of the preceding claims, **characterized in that** the sensor is directly fixed underneath the heating element of the cooking apparatus.

6. Cooking apparatus according to any of claims 1 to 5, **characterized in that** the sensor is secured on the securing part of the heating element, which extends parallel to the vertical walls of the vat.

7. Use of a device for the capacitive measurement of the quality and/or deterioration of a fluid that includes a sensor encapsulated in a perforated case and secured in the vat of a cooking apparatus, which includes a bottom, wherein said sensor is connected to an electronic processing circuit, wherein the sensor includes a pair of flat electrodes that each have the shape of a comb with a plurality of teeth, which are approximately parallel to each other and extend from a base, wherein the electrodes are arranged in relation to each other such that the teeth of one electrode fit between the teeth of the other electrode in approximately the same plane, **characterized in that** the encapsulated sensor is oriented in the vat such that the longitudinal axis of the base of each electrode extends parallel to the bottom of the vat and **in that** the plane of the sensor electrodes form an angle of between 0° and 60° with respect to the vertical direction.

8. Use of the measurement device according to claim 7, **characterized in that** the plane of the sensor electrodes forms an angle of between 0° and 30° with respect to the vertical direction.

9. Use of the measurement device according to claim 8, **characterized in that** the plane of the sensor electrodes forms an angle of approximately zero degrees with respect to the vertical direction.

10. Use of the measurement device according to any of claims 7 to 9, **characterized in that** the sensor is fixed in the vat in a removable manner on a securing support.

11. Use of the measurement device according to any of claims 7 to 10, **characterized in that** the sensor is directly fixed underneath the heating element of the cooking apparatus.
